# EUROPEAN PATENT APPLICATION

(11) **EP 2 260 848 A1**
(43) Date of publication of application: **15.12.2010**
(21) Application number: 09728694.2
(22) Date of filing: 30.03.2009
(51) Int. Cl.: A61K 31/496, A61P 27/02, A61P 31/04

(54) **AQUEOUS LIQUID CONTAINING GATIFLOXACIN, METHOD FOR PRODUCTION THEREOF, AND METHOD FOR PREVENTION OF PRODUCTION OF PRECIPITATES DURING STORAGE OF THE AQUEOUS LIQUID AT LOW TEMPERATURE OR UPON FREEZING/THAWING OF THE AQUEOUS LIQUID**

(30) Priority: 31.03.2008 US 41195 P
(71) Applicant: Kyorin Pharmaceutical Co., Ltd., Tokyo 101-8311 (JP); Senju Pharmaceutical Co., Ltd., Osaka-shi, Osaka 541-0046 (JP)
(72) Inventor: SAWA, Shirou, Kobe-shi Hyogo 651-2241 (JP)
(74) Representative: Gillard, Richard Edward
(86) International application number: PCT/JP2009/056473
(87) International publication number: WO 2009/123098

(57) **Abstract**

There is provided an aqueous liquid preparation comprising 0.65 to 2 w/v% of Gatifloxacin or a pharmacologically acceptable salt thereof or a hydrate thereof as free Gatifloxacin, and at least 0.5 w/v% of at least one of the ingredient selected from the group consisting of phosphoric acid, malonic acid, nicotinamide and a salt thereof, wherein a pH thereof is 5.8 to 6.9. In the aqueous liquid preparation, the solubility of Gatifloxacin is increased and the formation of a precipitate during storage at a lower temperature and at the time of freezing and thawing of the aqueous liquid preparation is suppressed by incorporating at least one of the ingredient selected from the group consisting of nicotinamide, caffeine, methylglucamine and Methyl parahydroxybenzoate into the aqueous liquid preparation.

## Description

### BACKGROUND OF THE INVENTION

### Technical Field

The present invention relates to a Gatifloxacin-containing aqueous liquid preparation, its production and a method for suppressing formation of a precipitate during storage at a lower temperature and at the same time of freezing and thawing of the aqueous liquid preparation.

### Background Art

Gatifloxacin is a new quinolone synthetic antimicrobial and exhibits strong antimicrobial activity against Gram-positive bacteria, anaerobic bacteria and mycoplasma, not to mention Gram-negative bacteria, and is marketed as eyedrops for treating bacterial conjunctivitis in the ophthalmologic field. It is known that the solubility of Gatifloxacin depends on a pH and the solubility around the physiological pH is very low.

U.S. Patent No. 6,333,045 discloses, as a technique of enhancing cornea permeability of a drug, increase in the solubility of a drug and suppression of precipitation of crystals, an aqueous liquid preparation containing Gatifloxacin or a salt thereof at a relatively low concentration of Gatifloxacin of lower than 0.5 w/v%, and sodium edetate at a low concentration.

Further, there are publications reporting that the use of sodium edetate causes problems eye irritation such as irritating to the cornea and the like during instillation (Pharmaceutical Research, 15, 8: 1275-1279, 1998; Drugs and the Pharmaceutical Sciences, 58, Ophthalmic Drug Delivery Systems: 188).

JP 10-7568 A discloses an aqueous composition having increased solubility of pyridone carboxylic acid, wherein a calcium salt and polyvinylpyrrolidone is added to pyridone carboxylic acid or a salt thereof with adjusting the pH 6.0 to 8.5, and also discloses that the solubility of pyridone carboxylic acid is further increased by further adding glycerin or boric acid to the aqueous composition.

JP 8-193024 A discloses an eyedrops composition, which comprises norfloxacin as an active ingredient and at least two acidic substances, one of which is boric acid, whose pH is in the range of 4.0 to 6.0.

JP 2002-2825 A discloses a liquid preparation of a quinolone antibiotic, wherein the quinolone antibiotic is dissolved in a solution of at least one phosphoric acid salt to adjust the pH to 5.5 to 7.5, with adjusting the osmotic pressure ratio to 0.85 to 1.20.

The disclosures of the above-mentioned documents are incorporated by reference herein.

### SUMMARY OF THE INVENTION

One object of the present invention is to increase the solubility of Gatifloxacin, thereby providing an aqueous liquid preparation containing Gatifloxacin at a high concentration, which is less irritating to the eyes and clear in a neutral pH region.

Another object of the present invention is to provide a process for producing such an aqueous liquid preparation.

Still another object of the present invention is to suppress the formation of a precipitate during storage at a lower temperature and at the time of freezing and thawing of an aqueous liquid preparation containing Gatifloxacin at a high concentration.

These and other objectives as well as advantages of the present invention will become apparent to those skilled in the art from the following description.

That is, the present invention relates to:
(1) An aqueous liquid preparation comprising 0.65 to 2 w/v% of Gatifloxacin or a pharmacologically acceptable salt thereof or a hydrate thereof as free Gatifloxacin, and at least 0.5 w/v% of at least one of the ingredient selected from the group consisting of phosphoric acid, malonic acid, nicotinamide and a salt thereof, wherein a pH thereof is 5.8 to 6.9;
(2) The aqueous liquid preparation according to the above (1), wherein the preparation further comprises at least 0.2 w/v% of sodium chloride;
(3) The aqueous liquid preparation according to the above (1) or (2), wherein the preparation further comprises at least one ingredient selected from the group consisting of nicotinamide, caffeine, methylglucamine, methyl parahydroxybenzoate and a salt thereof;
(4) A method for increasing the solubility of Gatifloxacin in an aqueous liquid preparation comprising Gatifloxacin or a pharmacologically acceptable salt thereof, or a hydrate thereof, which comprises incorporating at least 0.5 w/v% of at least one ingredient selected from the group consisting of phosphoric acid, malonic acid, nicotinamide and a salt thereof into the aqueous liquid preparation and adjusting the pH of the preparation to 5.8 to 6.9;
(5) A method for suppressing the formation of a precipitate during freezing and thawing of an aqueous liquid preparation comprising 0.65 to 2 w/v% of Gatifloxacin or a pharmacologically acceptable salt thereof, or a hydrate thereof as free Gatifloxacin, which comprises incorporating at least 0.5 w/v% of at least one ingredient selected from the group phosphoric acid, malonic acid, nicotinamide and a salt thereof and at least 0.2 w/v% of sodium chloride into the aqueous liquid preparation, and adjusting the pH of the preparation to 5.8 to 6.9;
(6) A method for suppressing the formation of a precipitate during storage at a low temperature and at the time of freezing and thawing of an aqueous liquid preparation comprising 0.65 to 2 w/v% of Gatifloxacin or a pharmacologically acceptable salt thereof, or a hydrate thereof as free Gatifloxacin, at least 0.5 w/v% of at least one ingredient selected from the group consisting of phosphoric acid, malonic acid, nicotinamide and a salt thereof and by at least 0.2 w/v% of sodium chloride, whose pH is 5.8 to 6.9, which comprises incorporating at least one ingredient selected from the group consisting of nicotinamide, caffeine, methylglucamine, methyl parahydroxybenzoate and a salt thereof into the aqueous liquid preparation; and
(7) A process for producing an aqueous liquid preparation comprising 0.65 to 2 w/v% of Gatifloxacin or a pharmacologically acceptable salt thereof, or a hydrate thereof as free Gatifloxacin, which comprises incorporating at least 0.5 w/v% of at least one ingredient selected from the group consisting of phosphoric acid, malonic acid, nicotinamide and a salt and adjusting the pH of the preparation to 5.8 to 6.9.

According to the present invention, the solubility of Gatifloxacin can be increased by incorporating at least one ingredient selected from the group consisting of phosphoric acid, malonic acid, nicotinamide and a salt thereof into an aqueous liquid preparation containing Gatifloxacin or a pharmacologically acceptable salt thereof, or a hydrate thereof, thereby making it possible to provide an aqueous liquid preparation containing Gatifloxacin at a high concentration, which is less irritating to the eyes and clear in a neutral pH region.

Further, according to the present invention, the solubility of Gatifloxacin can be increased by incorporating at least one ingredient selected from the group consisting of phosphoric acid, malonic acid, nicotinamide and a salt thereof into the aqueous liquid preparation containing Gatifloxacin or a pharmacologically acceptable salt thereof, or a hydrate thereof, thereby making it possible to provide an aqueous liquid preparation containing Gatifloxacin at a high concentration, which is less irritating to the eyes and clear in a neutral pH region, without addition of sodium edetate. Furthermore, according to the present invention, the increase in solubility of Gatifloxacin can be accelerated by incorporating at least one ingredient selected from the group consisting of phosphoric acid, malonic acid, nicotinamide and a salt thereof, and a small amount of sodium edetate into an aqueous liquid preparation containing Gatifloxacin or a pharmacologically acceptable salt thereof, or a hydrate thereof, thereby making it possible to provide an aqueous liquid preparation containing Gatifloxacin at a high concentration, which is less irritating to the eyes and clear in a neutral pH region.

Furthermore, according to the present invention, the formation of a precipitate in an aqueous liquid preparation containing Gatifloxacin during freezing and thawing can be suppressed by incorporating at least one ingredient selected from the group consisting of phosphoric acid, malonic acid, nicotinamide and a salt thereof, and sodium chloride into an aqueous liquid preparation containing Gatifloxacin.

Furthermore, according to the present invention, the formation of a precipitate in an aqueous liquid preparation containing Gatifloxacin during freezing and thawing can be suppressed by incorporating at least one ingredient selected from the group consisting of phosphoric acid, malonic acid, nicotinamide and a salt thereof, and sodium chloride without addition or with addition of a small amount of sodium edetate.

In addition, according to the present invention, the suppression of formation of a precipitate in an aqueous liquid preparation containing Gatifloxacin during freezing and thawing can be accelerated by incorporating at least one ingredient selected from the group consisting of phosphoric acid, malonic acid, nicotinamide and a salt thereof, and sodium chloride with addition of a small amount of sodium edetate.

Moreover, according to the present invention, the formation of a precipitate in an aqueous liquid preparation containing Gatifloxacin during storage at a low temperature and at the time of freezing and thawing can be suppressed by incorporating at least one ingredient selected from the group consisting of nicotinamide, caffeine, methylglucamine, methyl parahydroxybenzoate and a salt thereof into an aqueous liquid preparation comprising 0.65 to 2 w/v% of Gatifloxacin or a pharmacologically acceptable salt thereof, or a hydrate thereof as free Gatifloxacin, by at least 0.5 w/v% of at least one kind selected from phosphoric acid, malonic acid, nicotinamide and a salt thereof and at least 0.2 w/v% of sodium chloride, whose pH is 5.8 to 6.9.

### DETAILED DESCRIPTION OF THE PREFERRED EMBODIMENTS

The chemical name of Gatifloxacin is (1)-1-cyclopropyl-6-fluoro-1,4-dihydro-8-methoxy-7-(3-methyl-1-piperazinyl)-4-oxo-3-quinolinecarboxylic acid.

Examples of the pharmacologically acceptable salt of Gatifloxacin include a salt with an inorganic acid such as hydrochloric acid, sulfuric acid and phosphoric acid, a salt with an organic acid such as methanesulfonic acid, lactic acid, oxalic acid and acetic acid, or a salt of sodium, potassium, magnesium, calcium, aluminum, cerium, chromium, cobalt, copper, iron, zinc, platinum, silver or the like. Examples of the hydrate include 2/5, 1/2, 3/2, 5 hydrates and the like. The amount of Gatifloxacin or a pharmacologically acceptable salt thereof, or a hydrate thereof to be incorporated is 0.65 to 2 w/v%, preferably 0.7 to 1.5 w/v%, more preferably 0.7 to 1.1 w/v% in the liquid preparation in terms of free Gatifloxacin in view of stability (solubility, suppression of a precipitate) of the preparation.

Examples of the phosphoric acid, malonic acid, nicotinamide and a salt thereof include phosphoric acid, sodium dihydrogen phosphate, sodium hydrogen phosphate, trisodium phosphate, potassium dihydrogen phosphate, dipotassium phosphate, malonic acid, sodium malonate, potassium malonate, nicotinamide and the like. Further, hydrates thereof can also be used. These are used as a combination of one or two kinds or more. When it is used to increase the solubility of Gatifloxacin in the aqueous liquid preparation, the amount of the phosphoric acid, malonic acid or nicotinamide, or a salt thereof to be incorporated is at least 0.5 w/v%, preferably 0.6 to 3 w/v%, more preferably 0.7 to 2.2 w/v%, specifically preferably 0.7 to 1.2 w/v%.

The amount of the sodium chloride to be incorporated is at least 0.2 w/v%, preferably 0.2 to 1.8 w/v%, more preferably 0.2 to 1 w/v% in the liquid preparation.

The pH of the aqueous liquid preparation is generally from 5.8 to 6.9, preferably from 5.9 to 6.6.

The nicotinamide, caffeine, methylglucamine, methyl parahydroxybenzoate and a salt thereof can be used alone or by combining two or more thereof. Examples of a salt of methyl parahydroxybenzoate include sodium salt, potassium salt and the like. When it is used to suppress the formation of a precipitate during storage at a low temperature and the time of freezing and thawing of the aqueous liquid preparation, the amount of these to be incorporated is at least 0.01 w/v%, preferably 0.02 to 3 w/v%, more preferably 0.05 to 1 w/v%.

If necessary, isotonizing agents (potassium chloride, boric acid, glycerin, propyleneglycol, mannitol, sorbitol, glucose, etc.), preservatives (benzalkonium chloride, benzethonium chloride, chlorohexidine gluconate, chlorobutanol, benzyl alcohol, sodium dehydroacetate, parahydroxybenzoate esters, etc.), viscosity agents (methylcellulose, hydroxyethylcellulose, hydroxypropylmethylcellulose, carboxymethylcellulose, sodium hyaluronate, carboxyvinyl polymer, polyvinyl alcohol, polyvinyl pyrrolidone, macrogol, etc.), pH adjustors (hydrochloric acid, sodium hydroxide, acetic acid, phosphoric acid, etc.), stabilizers (sodium edetate, citric acid, etc.) and the like can be appropriately added to the aqueous liquid preparation of the present invention. Where sodium edetate is added, it is added preferably in an amount of 0.01 to 0.1 w/v%.

The aqueous liquid preparation of the present invention can be produced by a technique known in the art such as dispersion and dissolution of the desired ingredients in the form suitable for ophthalmic topical administration, preferably eyedrops.

In the present invention, "lower temperature" means temperature of around 4°C, preferably 4°C ± 1°C.

Hereinafter, the following Test Examples and Examples further illustrate the present invention in detail but are not to be construed to limit the scope thereof.

### Test Example 1: Solubility Test of Gatifloxacin

### (Test Procedure)

According to the formulations shown in Table 1, a saturated solution of Gatifloxacin containing the given amount of each additive was prepared. The solution containing sodium dihydrogen phosphate, boric acid or malonic acid was adjusted to a pH of 6.5 with hydrochloric acid and sodium hydroxide, and then stirred for 3 hours at room temperature. The solution containing nicotinamide was adjusted to 6.0 with hydrochloric acid and sodium hydroxide, filled in glass ampoules and shaken for 2 days at room temperature. As a control, a saturated solution of Gatifloxacin containing no additive was prepared, its pH was adjusted to 6.5 with hydrochloric acid and sodium hydroxide, and stirred for 3 hours at room temperature. Each solution was filtered through a membrane filter (0.45 µm), and the filtrate (1 mL) was precisely measured and a diluent was added thereto to precisely make its volume up to 50 mL. The resultant solution (3 mL) was precisely measured, an internal standard solution (3 mL) was precisely added thereto, and a diluent was added thereto to precisely make its volume up to 20 mL to prepare a sample solution. The concentration of Gatifloxacin was measured in the sample solution (20 µL) under the following HPLC conditions. The solubility was converted in terms of Gatifloxacin 3/2 hydrate.

### (HPLC measurement conditions)

Detector: Ultraviolet absorptiometer (measurement wavelength: 280 nm)
Column: Inertsil ODS-2.5 mm, 4.6 mmφ × 150 mm, GL Sciences Inc.
Column temperature: 40°C
Mobile phase: acetonitrile (180 mL), water (810 mL) and triethylamine (10 mL) were mixed, and the pH was adjusted to 4.5 with phosphoric acid.
Flow rate: 0.8 mL/min
Injection amount: 20 µL
Internal standard solution: a mobile phase solution of methyl p-aminobenzoate (1 → 10000)
Diluent: Mixture of acetonitrile, water and phosphoric acid (200 : 800 : 0.8)

### (Results)

The results are shown in Table 1.

**Table 1**

| Additives | Concentration of additive (w/v%) | Solubility of Gatifloxacin (w/v%) |
|---|---|---|
| None | 0 | 0.53 |
| Sodium dihydrogen phosphate dihydrate | 0.5 | 0.72 |
| | 1 | 0.91 |
| Malonic acid | 0.5 | 0.96 |
| | 1 | 0.93 |
| Nicotinamide | 1 | 1.41 |
| Boric acid | 0.5 | 0.58 |
| | 1 | 0.62 |

As shown in Table 1, the solubility of Gatifloxacin was increased by incorporating at least 0.5 w/v% of each of sodium dihydrogen phosphate, malonic acid and nicotinamide. On the other hand, the solubility was only about 0.6 w/v% when boric acid was incorporated by 1%.

### Test Example 2: Effect of Sodium Chloride on Suppression of Formation of Precipitate at the Time of Freezing and Thawing

### (Test Procedure)

According to the formulations shown in Table 2, aqueous liquid preparations containing Gatifloxacin of Example 1 and Comparative Examples 1 to 3 were prepared by a conventional method. Each aqueous liquid preparation was filled in glass ampoules and stored in a freezer at -30°C. The frozen aqueous liquid preparation was thawed at room temperature. The preparation in which a precipitate was formed was strongly shaken. These operations of freezing and thawing were repeated 10 times, and the properties was visually observed. According to the following criteria, the presence or absence of the formation of a precipitate was judged.

### (Judgment of Formation of Precipitate)

-: Foreign insoluble matter and change in the properties were not observed.
++: Foreign insoluble matter was remarkably formed.

**Table 2**

| Formulation (w/v%) | Example 1 | Comparative Example 1 | Comparative Example 2 | Comparative Example 3 |
|---|---|---|---|---|
| Gatifloxacin 3/2 hydrate | 0.75 | 0.75 | 0.75 | 0.75 |
| Sodium dihydrogen phosphate dihydrate | 2.0 | 2.0 | - | - |
| Boric acid | - | - | 2.0 | |
| Sodium citrate | - | - | - | 2.0 |
| Sodium chloride | 0.2 | - | 0.2 | 0.2 |
| Hydrochloric acid/sodium hydroxide | q.s. | q.s. | q.s. | q.s. |
| Purified water | q.s. | q.s. | q.s. | q.s. |
| pH | 6.5 | 6.5 | 6.5 | 6.5 |
| Formation of precipitate | - | ++ | ++ | ++ |

In Example 1 in which sodium dihydrogen phosphate and sodium chloride were incorporated, the formation of a precipitate at the time of freezing and thawing of the Gatifloxacin-containing aqueous liquid preparation was suppressed. On the other hand, in Comparative Example 1 in which sodium chloride was, the effect of suppressing the formation of a precipitate at the time of freezing and thawing was not observed. Further, in Comparative Example 2 in which boric acid was incorporated as a buffer and Comparative Example 3 in which sodium citrate was incorporated, the effect of suppressing the formation of a precipitate at the time of freezing and thawing was not observed even when sodium chloride was added.

### Test Example 3: Effect of pH on Suppression of Formation of Precipitate at the Time of Freezing and thawing

### (Test Procedure)

According to the formulations shown in Table 3, aqueous liquid preparations containing Gatifloxacin of Examples 2 and 3 and Comparative Examples 4 and 5 were prepared by a conventional method. Each aqueous liquid preparation was filled in glass ampoules and stored in a freezer at -30°C. The frozen aqueous liquid preparation was thawed at room temperature. The preparation in which a precipitate was formed was strongly shaken. These operations of freezing and thawing were repeated 10 times, and the properties were visually observed. According to the following criteria, the presence or absence of the formation of a precipitate was judged.

### (Judgment of Formation of Precipitate)

-: Foreign insoluble matter and change in the properties were not observed.
++: Foreign insoluble matter was remarkably generated.

**Table 3**

| Formulation (w/v%) | Example 2 | Example 3 | Comparative Example 4 | Comparative Example 5 |
|---|---|---|---|---|
| Gatifloxacin 3/2 hydrate | 0.75 | 0.75 | 0.75 | 0.75 |
| Sodium dihydrogen phosphate dihydrate | 0.8 | 0.8 | 0.8 | - |
| Sodium citrate | - | - | - | 0.8 |
| Sodium chloride | 0.2 | 0.2 | 0.2 | 0.2 |
| Hydrochloric acid/sodium hydroxide | q.s. | q.s. | q.s. | q.s. |
| Purified water | q.s. | q.s. | q.s. | q.s. |
| pH | 6.0 | 6.5 | 7.0 | 6.5 |
| Formation of precipitation | - | - | ++ | ++ |

The formation of a precipitate was suppressed at the time of freezing and thawing in the Gatifloxacin-containing aqueous liquid preparation by incorporating sodium dihydrogen phosphate dihydrate into the aqueous liquid preparation containing Gatifloxacin and adjusting the pH thereof to lower than 7. On the other hand, in the case where the pH was adjusted to 7 (Comparative Example 4) and the case where sodium citrate was added instead of sodium dihydrogen phosphate dihydrate (Comparative Example 5), a Foreign insoluble matter was remarkably generated at the time of freezing and thawing.

### Preparation Examples

According to the formulations shown in Table 4, Gatifloxacin-containing eyedrops are prepared by a conventional method.

**Table 4**

| Formulation (w/v%) | Example 6 | Example 7 | Example 8 | Example 9 | Example 10 |
|---|---|---|---|---|---|
| Gatifloxacin 3/2 hydrate | 0.75 | 0.75 | 0.75 | 1.0 | 1.0 |
| Sodium dihydrogen phosphate dihydrate | 0.8 | - | - | 1.0 | - |
| Malonic acid | - | 1.0 | - | - | - |
| Nicotinamide | - | - | 1.0 | - | 1.0 |
| Sodium chloride | 0.55 | 0.9 | 0.85 | 0.75 | 0.55 |
| Benzalkonium chloride | 0.005 | - | 0.005 | 0.005 | - |
| Sodium edetate | 0.01 | - | 0.01 | 0.01 | - |
| Hydrochloric acid/sodium hydroxide | q.s. | q.s. | q.s. | q.s. | q.s. |
| Purified water | q.s. | q.s. | q.s. | q.s. | q.s. |
| pH | 6.0 | 6.5 | 6.0 | 6.0 | 6.0 |

### Test Example 4: Low-temperature Storage Test and Freezing and Thawing Test

### (Preparation of Test Formulation)

Xanthan gum (6.25 g) was slowly added to purified water (2500 mL) at 25°C with stirring. The solution was warmed to 80°C and stirred for 5 hours while evaporated water was supplied every 1 hour. After completion of the stirring with warming, the solution was allowed to cool under room temperature. At the time when the temperature of the solution became 30°C or less, water was added to make the total volume up to 2500 mL. The solution was filtered through a membrane filter (5 µm) to obtain a 0.25% xanthan gum solution. Sodium chloride (11 g), sodium dihydrogen phosphate dihydrate (16 g), Gatifloxacin 3/2 hydrate (15 g) and 0.5% sodium edetate liquid (40 mL) were added to this 0.25% xanthan gum solution (1600 mL) and dissolved. A 0.2% benzalkonium chloride solution (50 mL) was added and dissolved. Purified water was added to make the total volume up to 1800 mL to prepare a solution containing Gatifloxacin. The solution containing Gatifloxacin (90 mL) was measured, the pH was adjusted to 6, and purified water was then added thereto to make the total volume up to 100 mL. The resulting solution was subjected to filtration sterilization with a membrane filter (0.22 µm), and 5 mL aliquots thereof were filled in polypropylene (PE) containers and polyethylene (PP) containers (Formulation 1). Further, 90 mL aliquots were measured, the additives were added and dissolved so that Formulations 2 to 13 as shown in Table 5 were obtained. followed by dissolving the mixtures. The resulting solution was adjusted to pH 6, made the total volume up to 100 mL by addition of purified water, and subjected to sterilized filtration with a membrane filter (0.22 µm). Five ml aliquots thereof were filled in polypropylene containers and polyethylene containers, respectively (Formulations 2 to 13).

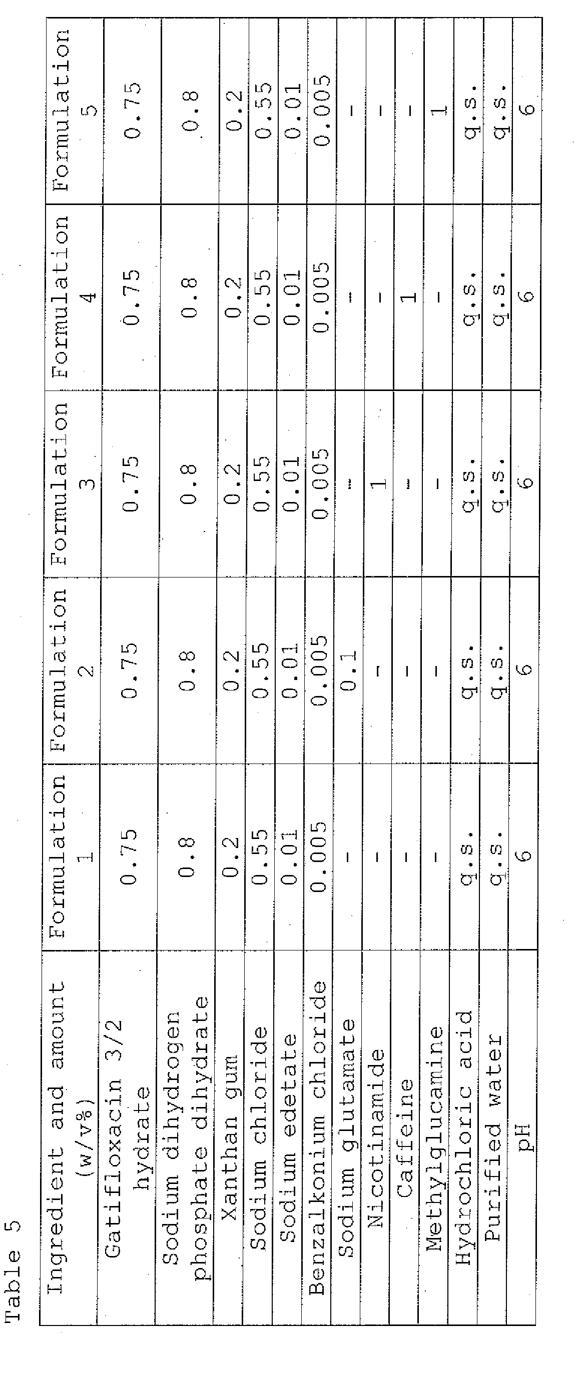

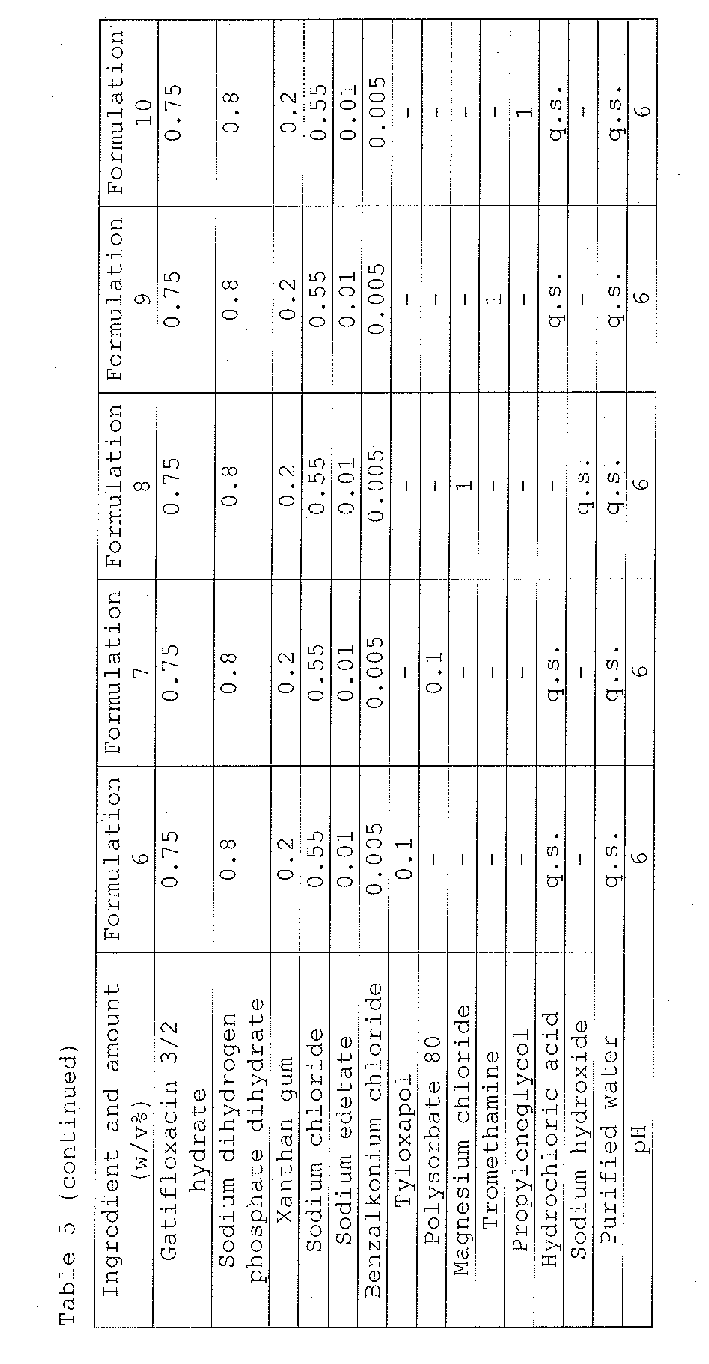

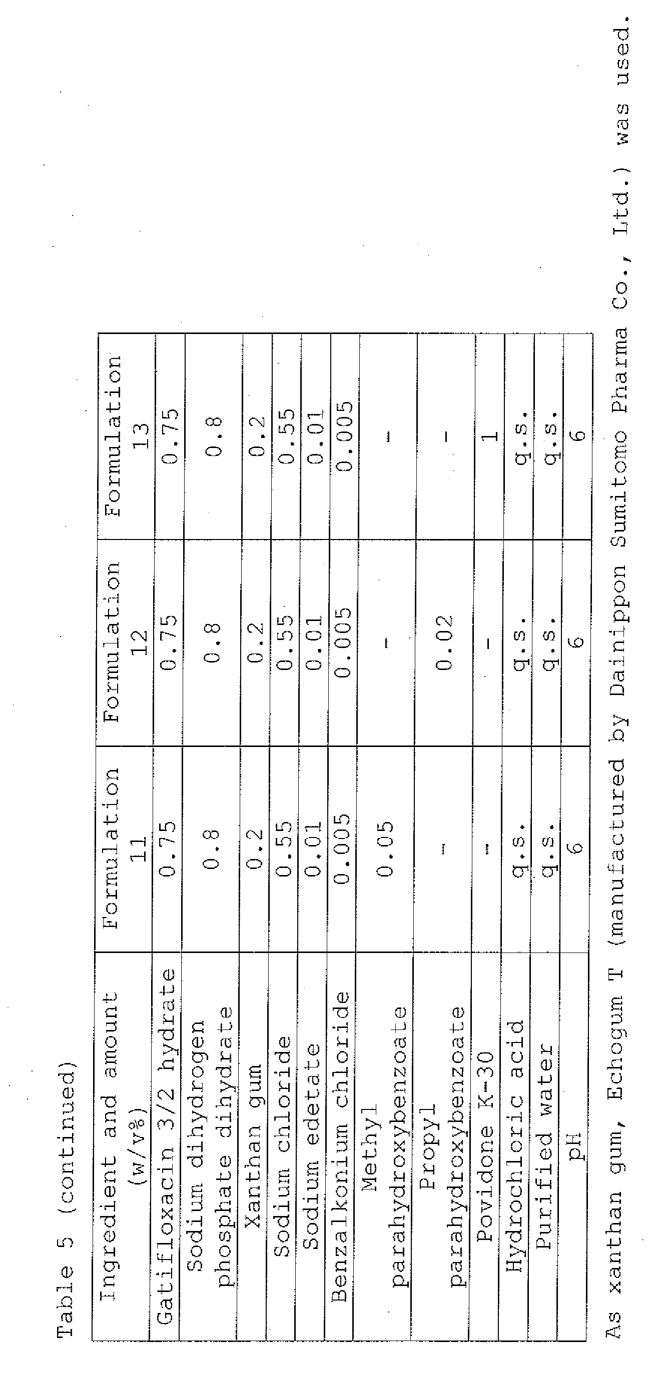

### (Test Procedure)

### (1) Low-temperature (4°C) storage test

Four samples of each of the Gatifloxacin-containing eyedrops of respective Formulations were stored at 4°C for 4 weeks and the description of the samples were visually observed. In accordance with the following criteria, the formation of a precipitate was judged and the number of (+) samples wherein formation of Foreign insoluble matter and description variation was observed was counted.

### (2) Freezing and thawing test

Three samples of each of the Gatifloxacin-containing eyedrops of respective Formulations were frozen at -30°C. Then, the samples were stored at 25°C to thaw the samples. These freeze-thawing procedures were repeated ten times and, after confirming that the sample completely thawed, the description was visually observed. In accordance with the following criteria, the formation of a precipitate was judged and the number of (+) samples wherein formation of Foreign insoluble matter and description variation was observed was counted.

### (Judgment of Formation of Precipitate)

-: Foreign insoluble matter and change in the properties were not observed.
+: Foreign insoluble matter and change in the properties were detected.

### (Results)

The results of the low-temperature (4°C) storage test are shown in Table 6.

**Table 6**

| Formulation No. | Vessel | Number of samples | Initial | After storage for 4 weeks |
|---|---|---|---|---|
| Formulation 1 | PP | 4 | 0 | 2 |
| | PE | 4 | 0 | 2 |
| Formulation 2 | PP | 4 | 0 | 3 |
| | PE | 4 | 0 | 2 |
| Formulation 3 | PP | 4 | 0 | 0 |
| | PE | 4 | 0 | 0 |
| Formulation 4 | PP | 4 | 0 | 0 |
| | PE | 4 | 0 | 0 |
| Formulation 5 | PP | 4 | 0 | 0 |
| | PE | 4 | 0 | 0 |
| Formulation 6 | PP | 4 | 0 | 2 |
| | PE | 4 | 0 | 4 |
| Formulation 7 | PP | 4 | 0 | 0 |
| | PE | 4 | 0 | 0 |
| Formulation 8 | PP | 4 | 0 | 4 |
| | PE | 4 | 0 | 4 |
| Formulation 9 | PP | 4 | 0 | 0 |
| | PE | 4 | 0 | 0 |
| Formulation 10 | PP | 4 | 0 | 0 |
| | PE | 4 | 0 | 0 |
| Formulation 11 | PP | 4 | 0 | 0 |
| | PE | 4 | 0 | 0 |
| Formulation 12 | PP | 4 | 0 | 2 |
| | | 4 | 0 | 1 |
| Formulation 13 | PE PP | 4 | 0 | 3 |
| | PE | 4 | 0 | 3 |

Table 7 shows the results of the freezing and thawing test.

**Table 7**

| Formulation No. | Vessel | Number of samples | Initial | After 10 cycles |
|---|---|---|---|---|
| Formulation 1 | PP | 3 | 0 | 1 |
| | PE | 3 | 0 | 1 |
| Formulation 2 | PP | 3 | 0 | 2 |
| | PE | 3 | 0 | 3 |
| Formulation 3 | PP | 3 | 0 | 0 |
| | PE | 3 | 0 | 0 |
| Formulation 4 | PP | 3 | 0 | 0 |
| | PE | 3 | 0 | 0 |
| Formulation 5 | PP | 3 | 0 | 0 |
| | PE | 3 | 0 | 0 |
| Formulation 6 | PP | 3 | 0 | 1 |
| | PE | 3 | 0 | 0 |
| Formulation 7 | PP | 3 | 0 | 2 |
| | PE | 3 | 0 | 3 |
| Formulation 8 | PP | 3 | 0 | 3 |
| | PE | 3 | 0 | 3 |
| Formulation 9 | PP | 3 | 0 | 3 |
| | PE | 3 | 0 | 3 |
| Formulation 10 | PP | 3 | 0 | 3 |
| | PE | 3 | 0 | 3 |
| Formulation 11 | PP | 3 | 0 | 0 |
| | PE | 3 | 0 | 0 |
| Formulation 12 | PP | 3 | 0 | 0 |
| | PE | 3 | 0 | 0 |
| Formulation 13 | PP | 3 | 0 | 3 |
| | PE | 3 | 0 | 2 |

As seen from the above results, it has been found that the formation of a precipitate during storage at a lower temperature (4°C) and at the time of freezing and thawing of the aqueous liquid preparation can be suppressed by addition of nicotinamide, caffeine, methylglucamine and Methyl parahydroxybenzoate to Gatifloxacin-containing eyedrops.

As described hereinabove, according to the present invention, the solubility of Gatifloxacin can be increased thereby making it possible to provide an aqueous liquid preparation containing Gatifloxacin or a pharmacologically acceptable salt thereof, or a hydrate thereof at a high concentration, which is less irritating to the eyes and clear in a neutral pH region. Further, the formation of a precipitate during storage at a lower temperature and at the time of freezing and thawing of the aqueous liquid preparation can be suppressed.

## Claims

1. An aqueous liquid preparation comprising 0.65 to 2 w/v% of Gatifloxacin or a pharmacologically acceptable salt thereof or a hydrate thereof as free Gatifloxacin, and at least 0.5 w/v% of at least one of the ingredient selected from the group consisting of phosphoric acid, malonic acid, nicotinamide and a salt thereof, wherein a pH thereof is 5.8 to 6.9.

2. The aqueous liquid preparation according to claim 1, wherein the preparation further comprises at least 0.2 w/v% of sodium chloride.

3. The aqueous liquid preparation according to claim 1 or 2, wherein the preparation further comprises at least one ingredient selected from the group consisting of nicotinamide, caffeine, methylglucamine and Methyl parahydroxybenzoate.

4. A method for increasing the solubility of Gatifloxacin in an aqueous liquid preparation comprising Gatifloxacin or a pharmacologically acceptable salt thereof, or a hydrate thereof, which comprises incorporating at least 0.5 w/v% of at least one ingredient selected from the group consisting of phosphoric acid, malonic acid, nicotinamide and a salt thereof into the aqueous liquid preparation and adjusting the pH of the preparation to 5.8 to 6.9.

5. A method for suppressing the formation of a precipitate during freezing and thawing of an aqueous liquid preparation comprising 0.65 to 2 w/v% of Gatifloxacin or a pharmacologically acceptable salt thereof, or a hydrate thereof as free Gatifloxacin, which comprises incorporating at least 0.5 w/v% of at least one ingredient selected from the group phosphoric acid, malonic acid, nicotinamide and a salt thereof and at least 0.2 w/v% of sodium chloride into the aqueous liquid preparation, and adjusting the pH of the preparation to 5.8 to 6.9.

6. A method for suppressing the formation of a precipitate during storage at a low temperature and at the time of freezing and thawing of an aqueous liquid preparation comprising 0.65 to 2 w/v% of Gatifloxacin or a pharmacologically acceptable salt thereof, or a hydrate thereof as free Gatifloxacin, at least 0.5 w/v% of at least one ingredient selected from the group consisting of phosphoric acid, malonic acid, nicotinamide and a salt thereof and by at least 0.2 w/v% of sodium chloride, whose pH is 5.8 to 6.9, which comprises incorporating at least one ingredient selected from the group consisting of nicotinamide, caffeine, methylglucamine and Methyl parahydroxybenzoate into the aqueous liquid preparation.

7. A process for producing an aqueous liquid preparation comprising 0.65 to 2 w/v% of Gatifloxacin or a pharmacologically acceptable salt thereof, or a hydrate thereof as free Gatifloxacin, which comprises incorporating at least 0.5 w/v% of at least one ingredient selected from the group consisting of phosphoric acid, malonic acid, nicotinamide and a salt and adjusting the pH of the preparation to 5.8 to 6.9.
